# EUROPEAN PATENT APPLICATION

(11) **EP 4 796 145 A1**
(43) Date of publication of application: **26.08.2026**
(21) Application number: 25159607.8
(22) Date of filing: 24.02.2025
(51) Int. Cl.: A61M 5/24, A61M 5/34, A61M 5/50, A61M 5/20, A61M 5/32

(54) **STATUS INDICATOR FOR MEDICAMENT CASSETTE**

(71) Applicant: Ypsomed AG, 3401 Burgdorf (CH)
(72) Inventor: Scheurer, Simon, 3006 Bern (CH); Glauser, Oliver, 3600 Thun (CH); Schüpbach, Simon, 3400 Burgdorf (CH); Wüthrich, Roman, 3700 Spiez (CH); Koch, Michelle, 4658 Dänliken (CH); Mower, Jimmy, Edinburgh, EH7 4BU (GB); James, Adam, Edinburgh, EH4 2HF (GB)

(57) **Abstract**

The present invention pertains to a cassette unit for a reusable or semi-disposable injection device comprising a reusable drive unit and a disposable cassette unit. The cassette unit is adapted to receive a medicament container and comprises indicators that indicate, in cooperation with a moveable part of the medicament container, if the cassette is in a used state or not.

## Description

### FIELD OF THE INVENTION

The present invention pertains to injection devices for self-administering medication, in particular to automated injection devices comprising a drive assembly to automatically inject a substance into the body of a user without the user having to apply an injection force. More precisely, the invention relates to a subassembly for such a device, the subassembly comprising a status indicator, allowing the user to immediately see if the subassembly is in a used or in an unused or new state.

### BACKGROUND OF THE INVENTION

Self-administration of therapeutic substances plays an increasingly important role in the treatment of many chronic medical conditions that require regular administration of a therapeutic agent, which can be a cost saving alternative to an administration by a health care professional. By way of example, chronic inflammatory conditions such as rheumatoid arthritis and Crohn's disease can be treated by regular subcutaneous injections of therapeutic agents, which can be performed by patients themselves. A further example are persons with diabetes mellitus, who require regular administration of insulin or insulin derivatives, which they need to dose and administer autonomously. A wide range of injection devices suitable for self-administration exist, however, due to practicality and hygiene, such devices are often designed as disposables and must be discarded after the contents have been dispensed. From an ecological and economical viewpoint such disposable devices are not ideal.

An attractive alternative is the use of reusable or semi-disposable devices, which comprise a disposable part on one hand, comprising hygiene and storage sensitive components, such as the therapeutic agent and the injection needle and a reusable part on the other hand, comprising expensive and non-sensitive parts that can be used multiple times. Examples of such devices have been disclosed in WO21254744 A1 and EP4275719 A2. Many such devices comprise a reusable drive module with a drive mechanism and control interfaces and a disposable cassette unit or cassette that comes with a pre-installed medicament container and a needle safety contraption.

A user of known devices might often have more than one such disposable cassette at home or at the site of administration, some of which may be used and are to be disposed and some of which may be unused and still contain one ore multiple doses of medicament. To make the usage more convenient for the user, it is desirable that the status of a cassette (i.e. if it is used or new) can be recognized immediately, e.g. by way of an appropriate marker or a status indicator. For example, if a patient has multiple cassette units stored at home and keeps the used cassettes for recycling, it may not immediately be obvious whether a given cassette unit is empty and used, or if it still contains medicament.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device or the part of the drug delivery device carrying an injection needle or an injection cannula, whereas the term "proximal" is meant to refer to the opposite direction or end pointing away from the needle or cannula.

### DESCRIPTION OF THE INVENTION

It is an objective of the invention to provide a status indicator for a disposable medicament cassette unit for a reusable or semi-disposable injection device, which allows a user to recognize immediately or at one glance if a cassette is empty and needs to be disposed, or if it is unused and/or still contains medicament.

This objective is achieved by the invention as described in the first independent claim. Further improvements and embodiments are detailed in the dependent claims.

The invention comprises indicators on the cassette unit that are to be aligned with a plunger of the medicament container when said container is empty and said cassette unit is thus in a used state or empty state and needs to be disposed of. The indicators may be located, arranged? on an outer surface of the cassette unit or they may be formed by the housing wall of the cassette unit or by any other part of the cassette unit.

The invention pertains to a cassette unit for a reusable injection device, which cassette unit may be coupled to a reusable drive unit to form an injection device. The cassette unit comprises an outer housing which is sleeve shaped, defines a longitudinal axis extending centrally along its long axis and comprises a proximal end and a distal end. The housing is adapted to at least radially envelop a medicament container. The proximal end is adapted to be inserted into and or/connected to a drive unit, wherein upon successful connection of the cassette unit to the drive unit, an operational injection device is formed. An opening on the proximal end of the housing allows parts of the drive or control mechanism of the drive unit to interact with or engage structures or parts inside the housing. A container holder is coaxially arranged radially inside the cassette unit housing and is adapted to laterally and axially fixate or support the medicament container. The distal ends of the housing and the container holder comprise openings through which an injection needle protrudes. Alternatively, the distal end of the container holder or the housing is adapted for connection of a disposable injection needle that must be attached by the user. In addition, the distal end of the cassette unit is adapted for attachment of a cassette cap, which is mounted in an initial or delivery state and must be removed by the user before an injection. The medicament container comprises an inner volume, radially defined by the side walls, which are preferably made from glass, and proximally confined by a plunger or stopper, preferably a rubber stopper, which is adapted to be moved in a distal direction along the longitudinal axis during a delivery or dispensing process, wherein the volume is continuously reduced and the liquid medicament is forced out of the container, through the injection needle, into the tissue of a patient or user. After the injection is terminated the plunger is in a distal end position and may contact or abut a distal end wall of the medicament container.

A window in the cassette unit provides visual access to the medicament container and to the plunger, such that a fill level or visual properties of the medicament may be inspected by the user. The window is in the form of a cutout or through hole in the housing wall and the container holder, which is coaxially arranged inside the housing. The missing material of the housing may be replaced by transparent plastic or glass. A rim or boundary of the window cutout in the housing may have an increased wall thickness compared to the rest of the housing wall. At least one window is formed on the cassette unit. In a preferred embodiment, there are two diametrically opposite windows on two opposite lateral sides of the cassette unit. The distal end position of the plunger is in an axial position such that it is visible through the at least one window. That is, at least a portion of the at least one window axially overlaps with the plunger end position. The distal end position of the plunger, relative to the housing or the container holder, is consistent for a given type of container and may thus be used as an indicator as to whether a cassette unit is in a used state and must be discarded. The drive unit of the injection device may comprise features or functions to prevent a cassette unit which is in the used state from being used again or from being able to trigger an injection process for more than one time. On a portion of the cassette unit housing or on any other part of the cassette unit, adjacent to the end position of the plunger, an indicator is placed or formed, which indicates said end position of the plunger. If the plunger is axially aligned with said indicator, the cassette unit is in a used state and may be discarded. The plunger being axially aligned is to be understood as at least a portion of the axial extension of the plunger overlapping with a portion of the axial extension of the indicator. For instance, a center of the indicator may be aligned with a proximal or a distal end of the plunger.

Alternatively, if the plunger or a portion of the plunger is located distally of the indicator in the axial direction, the plunger is also considered to be aligned with the indicator.

The indicator as claimed has the advantage of providing immediate certainty to a user whether a given cassette unit can still be used and/or still contains medicament or not. This is important in the case of many chronic diseases that require regular administration of medicament. In such cases a user or patient may have a stash or stock of medicament cassette units at home, e.g. stored in a refrigerator, some of which may be used, and may thus immediately see which cassette units are designated for recycling or disposal and which cassette units may still be used to inject medicament. In addition, the proposed solution with an indicator indicating a plunger end position can easily be integrated into existing devices and platforms, without any necessary device modifications or additional mechanisms. The proposed solution is only dependent on a plunger end position, which is consistent for a given container format and may vary only marginally between different types of containers. The indicator may thus be in a fixed axial position on the housing, irrespective of the medicament container used, or the indicators may be adapted to a given medicament container and its specific plunger end position relative to the housing.

In a preferred embodiment of the cassette unit, the at least one window of the cassette unit is elongated along the longitudinal axis. That is, an extension of the at least one window along the longitudinal axis is greater than an extension in the transversal direction. Preferably, the extension along the longitudinal axis is at least three times as great as the extension in the transversal direction. In the initial or delivery state, the plunger may be visible through the at least one window or not, depending on the specific type of medicament container used. However, in the end position, the plunger is always visible. The plunger end position is axially aligned with a distal portion of the at least one window, preferably with a distal end of the at least one window.

The indicator of the cassette unit may reside on an outer surface of the housing near or beside a distal end portion of the window cutout in the housing, such that the indicator is axially aligned with the plunger if it has reached the end position. By way of example, the indicator may be engraved or etched into the housing wall, such as by laser engraving or chemical etching. The indicator may further be printed onto the housing wall or onto a part that is attached or fixed to the housing wall.

In a preferred embodiment, the indicator is printed on a flexible label which is adhesively attached to the cassette unit housing wall. This has the advantage of easy customization in case the indicator is to be changed or adapted to a given medicament or container type. In a preferred embodiment, the indicator takes the form of broad lines or bars, extending circumferentially from both sides of the at least one window, wherein, in its end position, an upper or proximal edge of the plunger is aligned with an upper or proximal edge of the line. In another preferred embodiment, the indicator takes the form of arrows, arranged on both sides of the at least one window and pointing towards the plunger end position, visible through the at least one window, wherein in the end position, an axial center of the plunger is aligned with the tips of the arrows.

The cassette unit comprises a medicament container holder, adapted to axially and laterally support and fixate the medicament container, such that is stably held in the housing. In a preferred embodiment, the container holder is sleeve-shaped and extends coaxially to the housing, radially surrounding the medicament container. In some embodiments, the container holder or a separate part inside the container holder may comprise two tongs extending from a distal end thereof and being adapted to support a distal surface or shoulder of the medicament container. The tongs are flexibly mounted on the container holder and can radially flex outwards to allow a cap on a distal end of the container that covers the injection needle to pass through during installation from the proximal side of the container in the container holder.

Further, the indicator may be an integral part of the at least one window or the housing or of a part attached to the housing, such as a protrusion, a recess or any other geometric adaptation of the outer housing wall and/or the at least one window shape. The at least one window may for instance comprise slits or indents in the rim or fringe surrounding the window cutout in the housing wall, which indicate the plunger end position. Alternatively, protrusions on the rim or fringe, e.g. triangular protrusions in the form of arrows directed at a window center may serve as the indicators. In a preferred embodiment, the at least one window is split along the longitudinal axis into two sections. The at least one window may be split by the housing wall itself, e.g. by a transversal web or bridge serving as the indicator and being formed by the housing wall, extending from one side of the cutout in the housing wall to the opposite side. Alternatively, the at least one window may be split by the container holder, which is coaxially arranged inside the housing, wherein the web or bridge is formed from one side of the cutout in the container holder to the other side, serving as indicator. The distal section of the split window may have the form of an approximately circular window which has a diameter corresponding to an axial extension of the plunger. In this example, if the distal section of the window is at least partly filled out by the plunger and a proximal end of the plunger is at least approximately aligned with a distal end of the web or bridge, the plunger has reached the end position and is aligned with the indicators.

The medicament cassette unit may be usable for more than a single medicament delivery event, wherein the plunger of the medicament container is moved distally with each event or with each dose administered and the end position of the plunger is only reached after the final dose has been administered, i.e. when the container is empty. Preferably the cassette unit is adapted for single use or a single delivery event and the plunger end position is reached after the first and only delivery event. In any case, if the plunger end position has been reached, the medicament container is essentially empty. A small residue might remain in the container, e.g. in the injection needle or in the remaining space between the plunger and the distal end wall of the medicament container. In any case, if the plunger is in the distal end position, at least 95% of the initial contents of the medicament container have been dispensed. The cassette unit is thus in a used state and may be disposed of.

The injection device may be compatible with more than one type of medicament container. Examples of compatible container types include pre-filled syringes, on which an injection needle is non-removably pre-installed, or cartridges, which include an interface for attaching a disposable needle. Certain parts of the cassette unit may be changed or adjusted to a given container type, particularly the container holder and/or the distal part of the cassette unit housing, as well as the cassette cap. However, the interface to the drive unit remains unchanged. This allows for using cassette units with different containers to be used with a single drive unit. In addition, the majority of parts of the cassette unit remains unchanged when a different container is used, saving costs of development and redesign.

The cassette unit is adapted to be coupled to a reusable drive unit, upon which an operable injection device is formed. To this end, a proximal end of the cassette unit can be inserted into a distal opening of the reusable drive unit. The coupling may be achieved by a bayonet coupling, a releasable snap coupling or any other suitable means. In a preferred embodiment, the coupling is achieved by a lateral protrusion on the cassette unit, that can be engaged or clamped by a radially inwards directed clamping element of the drive unit. The lateral protrusion on the cassette unit, as well as the clamping structure may be chamfered or slanted, such that for coupling and decoupling the cassette and the drive unit, an initial coupling resistance or force must be overcome.

In a preferred embodiment, the indicators on the cassette unit housing are covered by a wall of the drive unit housing or by another element or part of the drive unit when the cassette unit is coupled to the drive unit, such that the indicators are only visible when the cassette unit is not coupled to the drive unit. The cassette unit, when coupled to the drive unit is radially surrounded or enveloped by a housing of the drive unit. The distal opening or bore of the drive unit for inserting the cassette unit may have a depth such that at least half of the length of the cassette unit extends into the bore and is thus radially covered by a part of the drive unit. Ideally, the drive unit comprises an axially elongated window, which aligns with the at least one window on the cassette unit, such that the medicament container and the plunger may still be visible if the cassette unit is coupled to the drive unit.

In a preferred embodiment of the cassette unit, the housing has the additional function of a needle protection sleeve which prevents inadvertent pricking or injury of a user by the injection needle. A distal end of the housing wall radially surrounds or envelops the injection needle in an initial or delivery state. To initiate an injection or delivery process, the user presses the distal end of the cassette unit onto the skin, upon which the sleeve-shaped housing moves proximally relative to the syringe holder and the medicament container, and the needle penetrates the skin. When a proximal triggering position is reached, the drive mechanism of the injection device is activated and starts the dispensing or delivery process. To this end, a proximal end of the housing is operably connected to or abuts a trigger element in the drive unit.

The housing may be locked in an initial state and may not be moveable into the proximal triggering position unless the cassette unit is coupled to the drive unit and the trigger mechanism or a blocking element of the drive unit or the cassette unit is released. The drive unit may comprise sensors or mechanisms adapted to detect if a cassette unit is in a used state or not and may only release the trigger element or the housing of the cassette unit if the cassette unit is not in a used state. After the plunger has reached the end position, the housing of the cassette unit can be moved back along the longitudinal axis into a distal locking position, in which a distal end of the housing extends beyond a distal end of the injection needle and from which the needle protection sleeve cannot be moved into the proximal direction anymore. In a preferred embodiment, the lockout or locking of the needle protection sleeve is enabled by distally extending arms of the drive unit that abut a proximally oriented stopping surface on the cassette unit housing. The cassette unit is now locked and in the used state and may be discarded.

The cassette unit as described is part of a modular injection device platform. The platform comprises the reusable drive unit as described and the cassette unit as described, wherein a ready-to-use injection device is formed by coupling the cassette unit to the drive unit. The cassette unit is disposable and preferably a new cassette must be coupled to the drive unit for each injection. In another embodiment, a cassette unit may be used multiple times, wherein each time a portion of the medicament contained in the medicament container is dispensed and a new sterile injection needle is attached to the cassette unit. The modular injection device platform may comprise other components such as cases or add-ons that provide additional functionality to the injection device.

The reusable drive unit to which the cassette unit can be coupled to, comprises a drive assembly, adapted to exert a distally directed force on the medicament plunger. The drive assembly further comprises an energy storage means and a mechanism to convert the stored energy into said distal force. The energy may be stored in a compression spring which can be released to drive a plunger rod against the plunger. In a preferred embodiment, the energy is chemically stored in a battery and is used by an electric motor to move or drive a plunger rod distally against the plunger. The drive unit may comprise user interfaces such as display or indication elements, buttons or switches which can be operated by a user.

For coupling the cassette unit to the drive unit, the cassette unit is inserted into a distal opening of the drive unit and moved proximally. The coupling is effectuated by a first engagement structure on the cassette unit which interacts with a second engagement structure on the drive unit. In a preferred embodiment, the first engagement structure has the form of a protrusion, and the second engagement structure has the form of a clamp, adapted to be engaged with or to grip said protrusion. The protrusion on the cassette unit is preferably arranged on a proximal end thereof and extends from the cassette unit housing radially outwards. The clamp is mounted to or comprises a radially pivotable part, that can pivot radially outwards against a biasing force. During coupling of the cassette unit and the drive unit, when the cassette unit is moved proximally after insertion into the distal opening, the protrusion abuts a distal end of the radially pivotable part of the clamp, upon which the clamp can radially deflect and engages the protrusion. To release the coupling, the user pulls the cassette unit distally and must overcome an initial engagement or coupling force between the clamp and the protrusion, upon which the protrusion is released from the clamp and the cassette unit can be removed. The drive unit further comprises a locking mechanism for locking the cassette unit in place when attached, such that it can no longer be decoupled by mere pulling of the cassette unit in distal direction by the user. The locking of the cassette unit to the drive unit is enabled by a radially pivotable arm with radially inwards extending protrusions that engages a recess or a distally oriented stopping surface of the cassette unit, thus preventing the cassette unit from moving in distal direction. In a preferred embodiment, the cassette unit comprises an NFC tag or NFC enabled label that can be scanned by an NFC reader in the drive unit to perform a medicament and safety check. By example, the check may comprise verifying the type and authenticity of a medicament and an expiration date of the medicament. Upon positive outcome of the check, the pivotable arm is caused to pivot or swivel inwards wherein the inwardly directed protrusions engage the recess or stopping surface and the cassette unit is locked to the drive unit. When the dispensing or injection process has been successfully completed, the cassette unit may be unlocked and can be pulled out of the drive unit. The dispensing process may only be triggered if the cassette unit is not in the used state, i.e. if there is medicament left to be dispensed. If the cassette unit is in the used state, triggering of the dispensing process may be prevented. Prevention of said triggering may be achieved by hindering the cassette housing or the trigger element from being moveable into the proximal triggering position or by cutting the operative connection of the trigger element to the drive assembly.

### BRIEF DESCRIPTION OF THE DRAWINGS

The subject matter of the invention will be explained in more detail in the following text with reference to preferred exemplary embodiments which are illustrated in the attached drawings, in which:
Figure 1 depicts a first embodiment of the cassette unit according to the invention, wherein the cassette unit is not in the used state;
Figure 2 depicts the first embodiment of the cassette unit of figure 1, wherein the cassette unit is in the used state;
Figure 3 depicts a second embodiment of the cassette unit according to the invention, wherein the cassette unit is not in the used state;
Figure 4 depicts the second embodiment of the cassette unit of figure 3, wherein the cassette unit is in the used state;
Figure 5 depicts a third embodiment of the cassette unit according to the invention, wherein the cassette unit is not in the used state;
Figure 6 depicts the third embodiment of the cassette unit of figure 6, wherein the cassette unit is in the used state; and
Figure 7 depicts an operational injection device comprising a drive unit and a cassette unit according to the invention.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

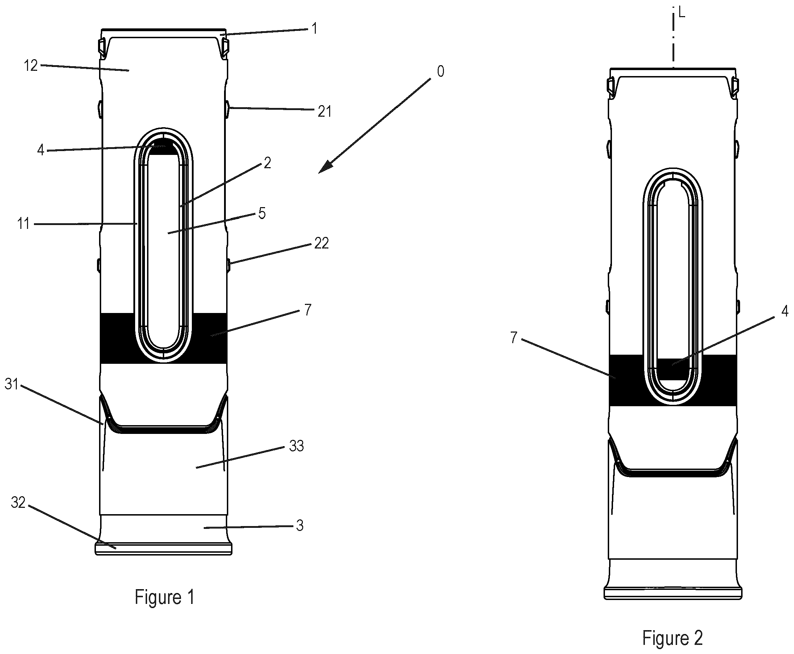

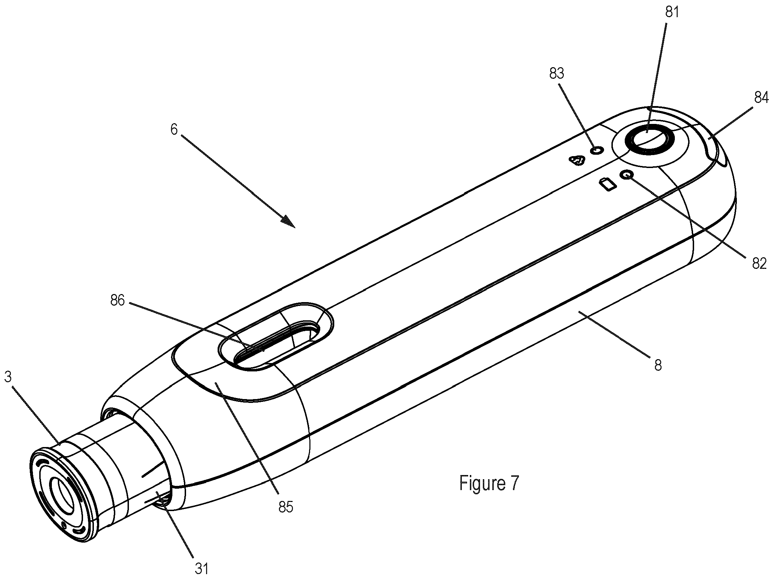

In Figure 1, a first embodiment of a cassette unit 0 according to the invention is depicted, wherein the cassette unit 0 is in an unused or new state. The cassette unit 0 comprises an outer housing 1 and a cap 3, attached to a distal end of the housing 1. Cap 3 comprises flexible holding tongues 31 which comprise radially inwardly directed recesses that are engaged in slanted, radially outwardly oriented protrusions (not shown) on the housing 1, leading to an initial peak in the removal force when a user pulls the cap 3 off in the distal direction. A distal, radially outwardly protruding rim 32 facilitates gripping and pulling of cap 3. An axially elongated window 5 is formed by a cut out in the housing 1 which is outlined by a boundary section, or a rim or fringe 11 of increased wall thickness. Coaxially and radially inside the housing 1 container holder 2 is arranged, which comprises a cutout of slightly smaller dimensions, such that a small portion of the container holder 2 is still visible along the boundary of window 5. A coupling bulge or protrusion 21 of the container holder 2 extends laterally through an opening in the cassette unit housing 1 on both sides, which bulge can be gripped by a clamp inside the drive unit 8 (visible in figure 7) to hold the cassette unit 0 in place. Locking arms 22 hold the housing 1 in place so that it cannot be moved in the proximal direction relative to the container holder 2 until, when the cassette unit 0 is coupled to the drive unit, the locking arms 22 are deflected radially inwards by an unlocking element of the drive unit. Window 5 provides visual access to the medicament container including plunger 4. A flexible label 12 is adhesively attached to an outer surface of housing 1 and may comprise printed information about the medicament and/or instructions for handling of the device. Additionally, in this embodiment the label 12 comprises a printed indicator 7, indicative of an end position of plunger 4. In this embodiment, the indicator 7 takes the form of a thick bar or line, extending from both lateral sides of window 5. A cap label 33 is adhesively attached to an outer surface of cap 3 and may match the label 12 in color and/or design, such that a given cap can easily be assigned to a corresponding cassette unit. In alternative embodiments, the cap label 33 may be of a different color and/or design such as to facilitate distinction from the label 12.

Figure 2 depicts the same first embodiment of the cassette unit 0 as figure 1, wherein the cassette unit 0 is now in a used state and the plunger 4 has moved along the longitudinal axis L into its end position. In this position, an upper or proximal boundary of plunger 4 is aligned with an upper or proximal boundary of the indicator 7.

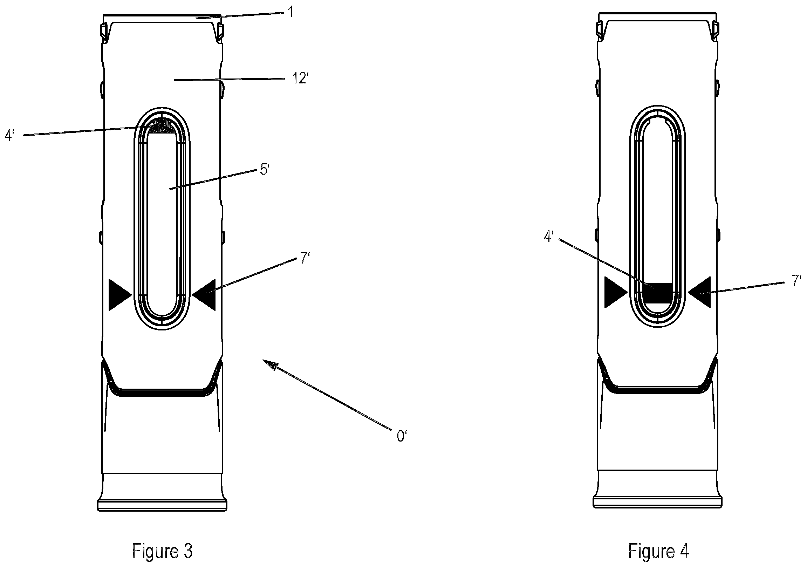

Figure 3 depicts a second embodiment of a cassette unit 0' according to the invention, wherein the cassette unit 0' is in a new or unused state. In this embodiment, all parts or elements are equal to the first embodiment, except for the label 12'. The indicator 7' is disposed on the label 12' and takes the form of radially inwardly directed arrows. In figure 4, a used state of the second embodiment of the cassette unit of Figure 3 is shown, wherein the cassette unit 0' is now in a used state and the plunger 4' is now aligned with the indicator 7', in particular, an axial center of plunger 4' is at least approximately axially aligned with the tip of the arrow-shaped indicator 7'.

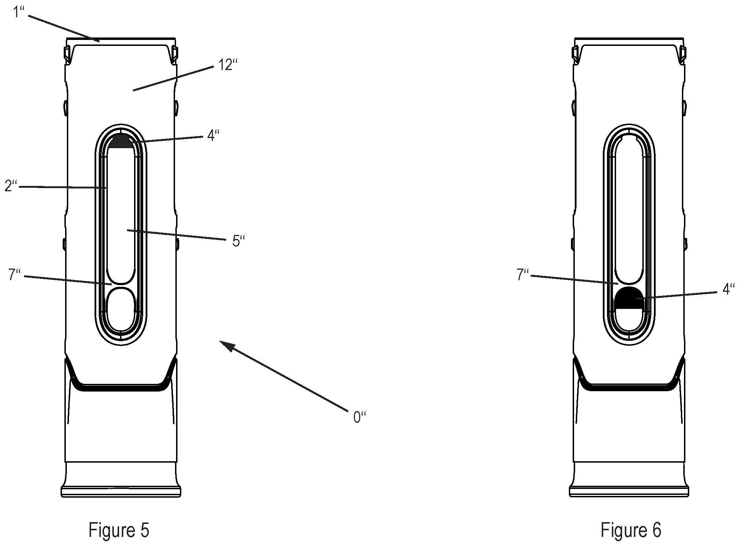

Figure 5 depicts a third embodiment of a cassette unit 0" according to the invention, wherein the cassette unit 0" is in a new or unused state. In this embodiment, the indicator 7" is not disposed on the housing 1", or on the label 12" but is instead formed by the container holder 2" as a bridge extending transversally across the window opening 5" in the syringe holder 2". All parts or elements except the container holder 2" are equal to the corresponding parts in the first and the second embodiment. In figure 6, the cassette unit of the third embodiment is shown in the used state, wherein the plunger 4" has reached the axial end position and is aligned with indicator 7".

Figure 7 depicts a complete or assembled injection device 6 comprising a cassette unit 0 according to the first embodiment of the invention, wherein the cassette unit 0 has been inserted into a reusable drive unit 8. The cap 3 of the cassette unit 0 protrudes from a distal end of the drive unit 8 and is held in place by the holding tongues 31. A button 81 and a battery indicator 82 as well as a connectivity indicator 83 are arranged on a proximal portion of the drive unit housing. A light strip 84 at a proximal end of the drive unit 8 gives information about the current status of the drive unit 8 such as an ongoing injection event or an error. The indicator 7 of the cassette unit 0 is not visible in this attached state, but is covered by a distal portion 85 of the housing of the drive unit 8. This is also the case for the second and third embodiments of the cassette units according to the invention and is caused by the fact that the window 86 of the drive unit 8 has a smaller axial extension than the window 5 of the cassette unit, wherein the proximal ends of both windows are axially aligned in the attached state.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF DESIGNATIONS

- L: longitudinal axis
- 0: cassette unit, first embodiment
- 1: cassette housing
- 11: window boundary
- 12: label
- 2: container holder
- 21: coupling bulge
- 22: locking arms
- 3: cassette cap
- 31: holding tongues
- 32: rim
- 33: cap label
- 4: plunger
- 5: window
- 6: injection device
- 7: indicator
- 8: drive unit
- 81: button
- 82: battery indicator
- 83: connectivity indicator
- 84: status indicator
- 85: distal housing section
- 86: window
- 0': cassette unit, second embodiment
- 1': cassette housing
- 12': label
- 4': plunger
- 5': window
- 7': indicator
- 0": cassette unit, third embodiment
- 1": housing
- 12": label
- 2": container holder
- 4": plunger
- 5": window
- 7": indicator

## Claims

1. A Cassette unit (0, 0', 0") for an injection device, which cassette unit (0, 0', 0") can be in a used state, the cassette unit (0, 0', 0") comprising:
- a medicament container, the medicament container comprising a plunger (4, 4', 4"),
- a sleeve-shaped housing (1, 1', 1"), enveloping the medicament container and defining a longitudinal axis (L),
- a container holder (2 ,2', 2"), arranged coaxially to and radially inside the housing (1, 1', 1") and adapted to axially and laterally fixate the medicament container,
- at least one window (5, 5', 5") through which the plunger (4, 4', 4") is visible, wherein the plunger (4, 4', 4") is moveable along the longitudinal axis (L) into a distal end position,
**characterized in that**:
the cassette unit (0, 0', 0") comprises at least one indicator (7 ,7', 7"), indicative of the distal end position of the plunger (4, 4', 4"), wherein, when the plunger (4, 4', 4") is axially aligned with the at least one indicator (7 ,7', 7"), the plunger (4, 4', 4")is in its distal end position and the cassette unit is in the used state.

2. The cassette unit (0, 0', 0") according to claim 1, wherein the at least one window (5, 5', 5") is formed by a cutout in a lateral wall of the housing (1, 1', 1") and a lateral wall of the container holder (2, 2', 2").

3. The cassette unit (0, 0', 0") according to claim 1 or 2, wherein the at least one window (5,5',5") is elongated along the longitudinal axis (L) and the plunger end position is near a distal end of the window (5,5',5").

4. The cassette unit (0, 0', 0") according to any of the previous claims, wherein the at least one indicator (7, 7', 7") resides on an outer surface of the housing (1, 1', 1").

5. The cassette unit (0, 0', 0") according to claim 4, wherein the at least one indicator (7, 7', 7") is etched or engraved onto the housing (1, 1', 1").

6. The cassette unit (0, 0', 0") according to claim 4, wherein the at least one indicator (7, 7') is printed onto a label (12, 12') which is adhesively attached to the housing (1, 1', 1").

7. The cassette unit (0, 0', 0") according to claim 1 to 3, wherein the at least one indicator (7, 7', 7")is formed by a web or bridge, extending transversally to the longitudinal axis (L) across the window (5, 5', 5"), wherein the web or bridge is formed by the housing (1") or by the container holder (2").

8. The cassette unit (0, 0', 0") according to any of the previous claims, wherein, when the cassette unit (0, 0', 0") is in the used state, at least 95% of the initial contents of the medicament container have been dispensed.

9. The cassette unit (0, 0', 0") according to any of the previous claims, wherein the medicament container is a pre-filled syringe comprising a fixedly pre-attached needle protruding from a distal end thereof.

10. The cassette unit (0, 0', 0") according to any of claims 1 to 8, wherein the medicament container is a pre-filled cartridge and wherein the container holder (2,2',2") comprises a threaded interface for threaded attachment of an injection needle.

11. The cassette unit (0, 0', 0") according to any of the previous claims, wherein the cassette unit (0,0',0") is disposable and can releasably be coupled to a reusable drive unit (8) containing a drive mechanism of the injection device, wherein the coupling is enabled by a clamp element on the drive unit (8) engaging a protrusion (21) on the container holder (2,2',2").

12. The cassette unit (0, 0', 0") according to claim 11, wherein the indicator (7, 7', 7") is covered by a housing of the drive unit (8) when the cassette unit (0, 0', 0") is coupled to the drive unit (8).

13. The cassette unit (0, 0', 0") according to claim 11 or 12, wherein the housing (1,1',1") is adapted to be operably connected to a trigger element of the drive unit and is moveable relative to the container holder (2,2',2") along the longitudinal axis (L) into a proximal triggering position.

14. The Cassette unit (0, 0', 0") according to one of claims 11 to 13, wherein, when the cassette unit (0, 0', 0") is in the used state, a proximal movement of the housing (1,1',1") is prevented by distally oriented arms (22) on the container holder (2,2',2"), which engage a proximally oriented stopping surface on the housing (1,1',1"), such that the drive mechanism of the drive unit (8) can only be triggered if the cassette unit (0, 0', 0") is not in the used state.

15. A modular injection device (6) comprising the cassette unit (0, 0', 0") according to one of the preceding claims coupled to a drive unit (8).
